# EUROPÄISCHE PATENTSCHRIFT

(11) **EP 0 610 350 B1**
(45) Veröffentlichungstag und Bekanntmachung des Hinweises auf die Patenterteilung: **03.01.1996**
(21) Anmeldenummer: 92922695.9
(22) Anmeldetag: 29.10.1992
(51) Int. Cl.: A61M 11/06, A61M 15/00

(54) **HANDVERNEBLER FÜR DAS ZERSTÄUBEN VON THERAPEUTISCHEN FLÜSSIGKEITEN**
HAND-HELD AEROSOL DISPENSER FOR THERAPEUTIC LIQUIDS
NEBULISEUR MANUEL POUR LA PULVERISATION DE LIQUIDES THERAPEUTIQUES

(30) Priorität: 29.10.1991 DE 9113446 U
(43) Veröffentlichungstag der Anmeldung: 17.08.1994
(73) Patentinhaber: KENDALL MEDIZINISCHE ERZEUGNISSE GmbH, D-93333 Neustadt (DE)
(72) Erfinder: HECKER, Karl-Heinz, D-8213 Aschau (DE)
(74) Vertreter: Rost, Jürgen
(86) Internationale Anmeldenummer: EP9202471
(87) Internationale Veröffentlichungsnummer: WO9308856

(56) Entgegenhaltungen:
- CH-A- 237 092
- FR-A- 2 437 839
- FR-E- 38 943
- US-A- 3 874 379
- US-A- 3 903 216
- US-A- 4 150 071
- US-A- 4 595 002

## Beschreibung

Die Erfindung betrifft einen Handvernebler mit den im Oberbegriff des Anspruchs 1 angegebenen Merkmalen.

Als Handvernebler ist ein vom Patienten in die Hand zu nehmendes Gerät für die Therapie zu Hause anzusehen. Bei dem Handvernebler gemäß dem DE-GM 90 11 768 ist der Vorratsbehälter für die zu zerstäubende Flüssigkeit ein integrierter Bestandteil des Gehäuses. Die Vorbereitung des Inhalierens durch den Patienten erfolgt dabei so, daß sich der Patent bisher eine für eine oder wenige Inhalationen notwendige Menge Medikament und Wasser mischt, in den Vernebler einbringt und mit der Inhalation beginnt.

Diese zeitaufwendige Vorarbeit bringt gerade bei Asthmatikern im Falle eines akuten Abfalls die Problematik mit sich, daß der Patient auf Grund der plötzlichen Atemnot, die sich explosionsartig steigert, in Panik gerät und dadurch noch weniger in der Lage ist, rasch und gezielt die Vorbereitungen für die notwendige Inhalation durchzuführen.

Als Alternative für diesen Fall kommt nur eine Notfall-Pulverinhalation in Frage, bei der aber in der Regel nur ein artverwandtes Medikament verwendet wird. Außerdem übt die trockene Pulverinhalation in den meisten Fällen einen starken Reiz auf die schon geschädigten Bronchien aus.

Außerdem wurde bisher sowohl eine Anfeuchtung des Bronchialbereichs als auch für die Herstellung einer Mischung aus Wasser und Medikament in der Regel einfaches Leitungswasser verwendet. Solches Wasser ist für die Inhalationstherapie denkbar ungeeignet, da es zwar bei äußerer und oraler Anwendung in der Regel zu keinen Komplikationen kommt, aber die im Leitungswasser enthaltenen Verunreinigungen m der Lunge zu schwerwiegenden Infekten führen können.

Ein Handvernebler gemäß dem Oberbegriff des Anspruchs 1 ist aus der US-PS 4 150 071 als bekannt zu entnehmen. Bei der bekannten Vorrichtung wird der Vorratsbehälter für die zu zerstäubende Flüssigkeit in das Gehäuse eingeschoben und durch eine Art von Paßsitz darin gehalten. Der Vorratsbehälter ist durch einen abnehmbaren Deckel verschließbar.

Bekannt ist aus der US-PS 4 595 002 ein Handvernebler, bei dem Teile durch eine Verschraubung miteinander verbunden sind.

Durch die US-PS 3 915 386 ist ein Raumbefeuchter bekannt geworden, bei dem ebenfalls mit einer Zerstäuberdüse gearbeitet wird. Solche Raumbefeuchter dienen dazu, das Raumklima in empfindlichen Bereichen, z.B. in Operationssälen, zu optimieren Bei dem bekannten Gerät ist ein von unten in das Gerät einschraubbarer Vorratsbehälter vorgesehen, der sterilisiertes Wasser enthalten kann. Als Handgerät ist die bekannte Vorrichtung nicht geeignet, weil der Vorratsbehälter ein Fassungsvermögen von etwa 1 Liter hat, und es ist auch nicht vorgesehen, eine Medikamentenmischung zu zerstäuben, was bei einer Raumbefeuchtung auch nicht angebracht wäre.

Der Erfindung liegt die Aufgabe zu Grunde, den Handvernebler der im Oberbegriff des Anspruchs 1 als bekannt vorausgesetzten Art so weiterzubilden, daß ein ständig betriebsbereiter Handvernebler mit optimalen Zerstäubungsergebnissen geschaffen wird, bei dem die Verwendung von verunreinigtem Wasser ausgeschlossen ist.

Diese Aufgabe wird mit den im Anspruch 1 angegebenen Merkmalen gelöst.

Mit der Erfindung wird ein Handvernebler zur Verfügung gestellt, bei dem in einem vorn Gehäuse trennbaren Vorratsbehälter Sterilwasser oder eine Mischung aus Sterilwasser und Medikament zur Verfügung gestellt wird, wobei zur Verbindung zwischen Vorratsbehälter und Gehäuse ein gegenüber den Abmessungen des Vorratsbehälters reduzierter Schraubverschluß zur Anwendung gelangt.

Dadurch wird ermöglicht, daß ein bereits vorgemischtes Medikament in den Vorratsbehälter abgefüllt wird, so daß der Patient nur den Vorratsbehälter in einfacher und sicherer Weise mit dem Gehäuse verbinden muß, um einen betriebsbereiten Vernebler zu erhalten.

Der nach unten bis in Bodennähe des Vorratsbehälters reichende Saugrüssel sorgt dafür, daß der Vorratsbehälter nahezu restlos entleert werden kann.

Vorteilhafte weitere Ausführungsformen sind Gegenstand der Ansprüche 2 bis 10.

Besonders vorteilhafte Verneblungsergebnisse, und zwar insbesondere hinsichtlich der Trennung der kleinen Tröpfchen von den größeren, können dadurch erzielt werden, daß die Zerstäuberdüse von einer mit zeitlichem Abstand innerhalb des Gehäuses angeordneten, zylindrischen, unten offenen Trennkammer umgeben ist, welche über Öffnungen in der Zylinderwand und über Öffnungen in der Gehäusewand mit der Außenluft in Verbindung steht. Auf diese Weise wird der erzeugte Nebel wie in einem Schornstein nach unten geführt, wobei durch die dann erfolgende Umlenkung um etwa 180° eine besonders gute Trennung der kleinen Tröpfchen von den größeren Tröpfchen erreicht wird, die infolge der Zentrifugalkraft an die Innenwand des Gehäuses geschleudert werden und dann in den Vorratsbehälter zurückfließen.

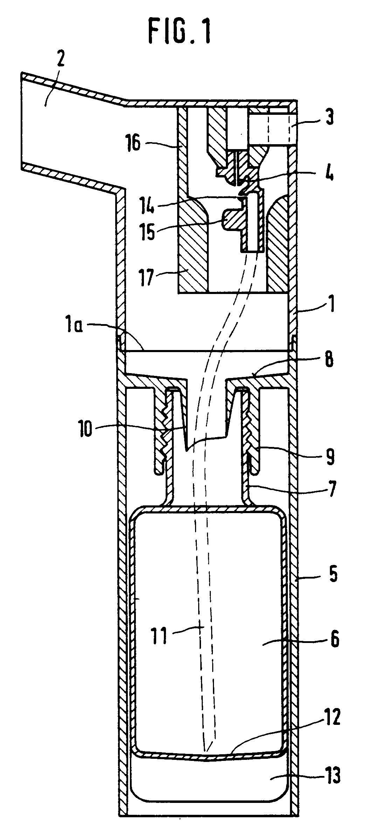

Im folgenden wird die Erfindung anhand eines in der Zeichnung dargestellten Ausführungsbeispiels näher erläutert. Es zeigt
- Fig. 1: einen Längsschnitt durch den Handvernebler,
- Fig. 2: eine Seitenansicht des Vorratsbehälters,
- Fig. 3: den in Fig. 2 gezeigten Vortatsbehälter von unten.

Das Gehäuse 1, welches längs einer Fuge 1a zweigeteilt ist, weist wie üblich ein Mundrohr 2, eine Druckluftzuleitung 3 und eine Zerstäuberdüse 4 auf.

Das Gehäuse 1 hat einen nach unten ragenden, unten offenen Zylinderteil 5. Innerhalb dieses Zylinderteils 5 ist ein Vorratsbehälter 6 angeordnet.

Der Vorratsbehälter 6 ist lösbar mit dem Gehäuse 1 dadurch verbunden, daß ein Halsteil 7 des Vorratsbehälters 6 in einen von einer Zwischenwand 8 nach unten vorstehenden Stutzen 9 mit Innengewinde eingeschraubt ist. Innerhalb des Stutzens 9 befindet sich ein Aufbrechstutzen 10, mit dem beim Einschrauben des Vorratsbehälters 6 sein Deckel aufgebrochen wird.

Der Innendurchmesser dieses Aufbreckstutzens 10 ist größer als der Außendurchmesser eines Saugrüssels 11, damit in den Vorratsbehälter 6 Luft eintreten kann und auch die durch die Trennvorrichtung separierten größeren Tröpfchen in den Vorratsbehälter 6 zurückfließen können. Um dieses Zurückfließen zu erleichtern, hat die Zwischenwand 8 ein allseitiges Gefälle zu dem Aufbrechstutzen 10.

Der Saugrüssel 11, der oben an einen in der Ansaugöffnung 14 ausmündenden Ansaugkanal angeschlossen ist, ist bis in die Nähe des Bodens 12 des Vorratsbehälters 6 geführt, damit dieser möglichst restlos entleert werden kann. Zu diesem Zweck ist es vorteilhaft, daß der Boden 12 des Vorratsbehälters 6 allseitig zu einem Tiefpunkt abfällt.

An seiner Unterseite trägt der Boden 12 des Vorratsbehälters 6 einen Steg 13, welcher als Handhabe beim Einschrauben des Vorratsbehälters 6 verwendet wird.

Im oberen Teil des Gehäuses 1 befindet sich die Einrichtung zum Zerstäuben der Flüssigkeit und zum Trennen der kleinen Tröpfchen von den nicht brauchbaren größeren Tröpfchen. Diese Einrichtung besteht aus einer Zerstäuberdüse 4, welcher von oben her über die Druckluftzuleitung 3 Druckluft zugeführt wird. Bei Austritt der Druckluft aus der Zerstäuberdüse 4 wird durch das Venturi-Prinzip aus der Ansaugöffnung 14 Flüssigkeit angesaugt und vor allem dadurch zerstäubt, daß der austretende Druckluftstrahl mit den Flüssigkeitströpfchen auf einen Prallkörper 15 auftrifft.

Diese Einrichtung ist innerhalb einer zylindrischen Trennkammer 16 untergebracht. Diese Trennkammer 16 ist mit Abstand exzentrisch innerhalb des Gehäuses 1 angeordnet. Der Innenraum steht über in der Wand der Trennkammer 16 und in der Wand des Gehäuses 1 vorgesehene Öffnungen (nicht gezeichnet) mit der Außenluft in Verbindung. Auf diese Weise wird unter dem Einfluß des in der Trennkammer eintretenden Unterdrucks Außerluft angesaugt und wie in einem Kamin zusammen mit dem Nebel nach unten geführt. Bei der am unteren Ende der Trennkammer erfolgenden Umlenkung dieses Gemischs werden die größeren Tröpfchen, welche für die Inhalation nicht brauchbar sind, weggeschleudert und fließen in den Vorratsbehälter 6 zurück.

Innerhalb der Trennkammer 16, und zwar in deren unterem Teil, ist eine Verdickung 17 mit geringerem Innendurchmesser als der übrige Kammerbereich vorgesehen, welche oben mit einer Abrundung etwa in Höhe der Ansaugöffnung 14 endet. Diese Verdickung dient ebenfalls der Optimierung der Trennung der kleinen von den größeren Tröpfchen.

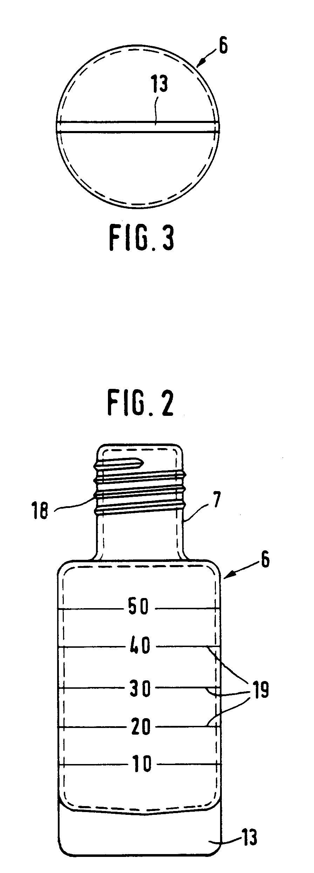

In Fig. 2 ist ein Vorratsbehälter 6 dargestellt, welcher etwa flaschenförmig ist und auf seinem Halsteil 7 mit einem Gewinde 18 versehen ist.

Der Vorratsbehälter 6 ist mit Markierungen 19 zum Anzeigen des Füllungsgrades versehen. Diese Markierungen 19 können beispielsweise dann verwendet werden, wenn die Mischung Sterilwasser / Medikament vom Patienten selbst hergestellt wird. Die Markierungen 19 erleichtern das Herstellen bestimmter Mischungsverhältnisse, sie zeigen aber auch an, für wieviel Inhalationen der Inhalt des Vorratsbehälters noch ausreicht. Dabei ist der Vortatsbehälter 6 normalerweise vor dem Mischen in das durchsichtige Gehäuse eingeschraubt.

## Patentansprüche

1. Handvernebler für das Zerstäuben von therapeutischen Flüssigkeiten bei der Inhalationstherapie mit einer in einem Gehäuse angeordneten Zerstäuberdüse (4) für den Austritt von Druckluft und mit einem Vorratsbehälter (6) für die Flüssigkeit, aus dem nach dem Venturi-Prinzip durch eine im Bereich der Zerstäuberdüse ausmündende Ansaugöffnung (14) die Flüssigkeit angesaugt und unter Verwendung eines der Zerstäuberdüse (4) gegenüberliegenden Prallkörpers (15) in feinste Teilchen zerstäubt wird, wobei der in seinen Abmessungen mit den Abmessungen des Gehäuses (1) entsprechende Vorratsbehälter (6) lösbar im unteren Teil des Gehäuses (1) untergebracht ist, der im Bereich der Zerstäuberdüse (4) ausmündende Kanal nach unten durch einen Saugrüssel (11) verlängert ist, der durch eine obere Öffnung des Vorratsbehälters (6) bis in die Nähe des Bodens (12) des Vorratsbehälters (6) geführt ist und im Vorratsbehälter (6) sich Sterilwasser oder eine Mischung aus Sterilwasser und Medikament befindet,
**dadurch gekennzeichnet,**
daß eine im Gehäuse (1) angeordnete Zwischenwand (8) einen nach unten vorstehenden, mit Innengewinde versehenen Stutzen (9) trägt, in welchen ein Halsteil (7) des Vorratsbehälters (6) eingeschraubt ist.

2. Handvernebler nach Anspruch 1, dadurch gekennzeichnet, daß innerhalb des Stutzens (9) ein Aufbrechstutzen (10) angeordnet ist, dessen Außenabmessung kleiner als die Innenabmessung des Halsteils (7) ist.

3. Handvernebler nach Anspruch 1 oder 2, dadurch gekennzeichnet, daß sich der Vorratsbehälter (6) zumindest teilweise innerhalb eines vom Gehäuse (1) nach unten ragenden Zylinderteils (5) befindet.

4. Handvernebler nach einem der Ansprüche 1 bis 3, dadurch gekennzeichnet, daß der Boden (12) des Vortatsbehälters (6) allseitig zu einem Tiefpunkt abfällt.

5. Handvernebler nach einem der Ansprüche 1 bis 4, dadurch gekennzeichnet, daß unter dem Boden (12) des Vorratsbehälters (6) ein radialer Steg (13) als Handhabe für das Einsetzen des Vortatsbehälters (6) angeordnet ist.

6. Handvernebler nach einem der Ansprüche 1 bis 5, dadurch gekennzeichnet, daß der Vortatsbehälter (6) mit den Füllungsgrad anzeigenden Markierungen (19) versehen ist.

7. Handvernebler nach einem der Ansprüche 1 bis 6, dadurch gekennzeichnet, daß die Zerstäuberdüse (4) von einer mit seitlichem Abstand innerhalb des Gehäuses (1) angeordneten, zylindrischen, unten offenen Trennkammer (16) umgeben ist, welche über Öffnungen in der Zylinderwand und über Öffnungen in der Gehäusewand mit der Außenluft in Verbindung steht.

8. Handvernebler nach Anspruch 7, dadurch gekennzeichnet, daß die Innenwand der Trennkammer (16) im unteren Teil mit einer zylindrischen, oben abgerundeten Verdickung (17) versehen ist, welche einen geringeren Innendurchmesser als der übrige. Kammerbereich aufweist.

9. Handvernebler nach Anspruch 8, dadurch gekennzeichnet, daß der obere Rand der Verdickung (17) etwa in Höhe der Ansaugöffnung (14) angeordnet ist.

10. Handvernebler nach einem der Ansprüche 7 bis 9, dadurch gekennzeichnet, daß die Trennkammer (16) exzentrisch innerhalb des Gehäuses (1) angeordnet ist.

## Claims

1. Hand-held aerosol dispenser for therapeutic liquid for inhalation therapy having a nebulizer nozzle (4) arranged in a housing for the exit of compressed air, and with a storage container (6) for the liquid, wherein according to the Venturi-principle the liquid is taken in from the storage container (6) by means of a suction aperture (14) ending in a region of the nebulizer nozzle and is nebulized into very fine particles using an impact member (15) opposing the nebulizer nozzle (4), said storage container (6) corresponding in its dimensions to the size of the housing (1) is removably arranged in the lower region of the housing (1), wherein the channel ending in the region of the nebulizer nozzle (4) is extended downwardly by a suction tube (11), which extends through an upper aperture of the storage container (6) to the vicinity of the bottom (12) of the storage container (6), and wherein sterile water or a mixture of sterile water and medication is contained in the storage container (6)
**characterized in that**
a partition (8) arranged in the housing (1) supports a female threaded connecting part (9) protruding downwardly, into which a neck section (7) of the storage container (6) is screwed.

2. Hand-held aerosol dispenser according to claim 1, characterized in that a fracturing connection part (10) is arranged within the connection part (9), whereby its outer dimensioning is smaller than the inner dimensioning of the neck section (7).

3. Hand-held aerosol dispenser according to claim 1 or 2, characterized in that the storage container (6) is at least partially located within a cylinder part (5) extending downwardly from the housing (1).

4. Hand-held aerosol dispenser according to claims 1 through 3, characterized in that the bottom (12) of the storage container (6) slopes in every direction to a low point.

5. Hand-held aerosol dispenser according to one of the claims 1 through 4, characterized in that a radial rib (13) is arranged below the bottom (12) for handling the insertion of the storage container (6).

6. Hand-held aerosol dispenser according to one of the claims 1 through 5, characterized in that the storage container (6) is provided with markings (19) indicating the level of filling.

7. Hand-held aerosol dispenser according to one of the claims 1 through 6, characterized in that the nebulizer nozzle (4) is surrounded by a cylindrical partitioning chamber (16) which is open at the bottom and which is arranged at a lateral distance within the housing (1), wherein the partitioning chamber (16) by means of apertures in the cylindrical wall and by means of apertures in the wall of the housing is in communication with the outside air

8. Hand-held aerosol dispenser according to claim 7, characterized in that the inner wall of the partitioning chamber (16) is provided at its lower section with a cylindrical thickened section (17) which is rounded on the top, and which has a smaller inner diameter than the other chamber regions.

9. Hand-held aerosol dispenser according to claim 8, characterized in that the upper edge of the thickened section (17) is approximately arranged at the level of the suction' aperture (14).

10. Hand-held aerosol dispenser according to the claims 7 through 9, characterized in that the partitioning chamber (16) is arranged eccentrically within the housing (1).

## Revendications

1. Atomiseur à main pour l'atomisation de liquides thérapeutiques lors de la thérapie par inhalation avec une buse d'atomisation (4) disposée dans un boîtier pour la sortie d'air comprimé et avec un réservoir (6) pour le liquide, hors duquel le liquide est aspiré selon le principe Venturi à travers un orifice d'aspiration (14), qui aboutit proche de ladite buse d'atomisation, et est atomisé sous forme de fines particules au moyen d'un corps de rebondissement (15) qui fait face à la buse d'atomisation (4); le réservoir (6), dont les dimensions correspondent aux dimensions du boîtier (1), étant disposé de façon détachable dans la partie inférieure du boîtier (1); le conduit, qui aboutit proche de la buse d'atomisation (4), étant allongé vers le bas par un tuyau d'aspiration (11), qui passe par un orifice supérieur du réservoir (6) jusque proche du fond (12) du réservoir (6), et dans le réservoir (6) se trouvant de l'eau stérilisée ou un mélange d'eau stérilisée et d'un médicament,
**caractérisé** en ce que
une paroi intermédiaire (8) disposée dans le boîtier (1) comporte un manchon (9) qui est pourvu d'un taraudage et est dirigé vers le bas, et dans lequel est vissé un col (7) du réservoir (6).

2. Atomiseur à main selon la revendication 1, caractérisé en ce qu'un manchon d'enfoncement (10), dont la dimension extérieure est plus petite que la dimension intérieure du col (7), est disposé à l'intérieur du manchon (9).

3. Atomiseur à main selon la revendication 1 ou 2, caractérisé en ce que le réservoir (6) se trouve au moins partiellement à l'intérieur d'une partie cylindrique (5) se dirigeant vers le bas à partir du boîtier (1).

4. Atomiseur à main selon l'une des revendications 1 à 3, caractérisé en ce que le fond (12) du réservoir (6) descend de toutes parts vers un point bas.

5. Atomiseur à main selon l'une des revendications 1 à 4, caractérisé en ce qu'une traverse (13) radiale est disposée sous le fond (12) du réservoir (6) en tant que prise pour l'installation du réservoir (6).

6. Atomiseur à main selon l'une des revendications 1 à 5, caractérisé en ce que le réservoir (6) est pourvu de marques (19) indiquant le taux de remplissage.

7. Atomiseur à main selon l'une des revendications 1 à 6, caractérisé en ce que la buse d'atomisation (4) est entourée par une chambre de séparation (16) cylindrique, qui est ouverte vers le bas et qui est disposée à l'intérieur du boîtier (1) avec une distance latérale, la chambre de séparation (16) se trouvant en contact avec l'air extérieur par des orifices dans la paroi du cylindre et par des orifices dans la paroi du boîtier.

8. Atomiseur à main selon la revendication 7, caractérisé en ce que la paroi intérieure de la chambre de séparation (16) est pourvue dans la partie inférieure d'un épaississement (17) cylindrique et arrondi vers le haut et ayant un diamètre intérieur plus petit que la partie restante de la chambre.

9. Atomiseur à main selon la revendication 8, caractérisé en ce que le bord supérieur de l'épaississement (17) se trouve à peu près à la hauteur de l'orifice d'aspiration (14).

10. Atomiseur à main selon l'une des revendications 7 à 9, caractérisé en ce que la chambre de séparation (16) est disposée de façon excentrique à l'intérieur du boîtier (1).
